# EUROPÄISCHE PATENTSCHRIFT

(11) **EP 1 723 985 B1**
(45) Veröffentlichungstag und Bekanntmachung des Hinweises auf die Patenterteilung: **12.03.2008**
(21) Anmeldenummer: 05010846.3
(22) Anmeldetag: 19.05.2005
(51) Int. Cl.: A61N 2/02

(54) **Magnetfeldtherapiegerät zum Erzeugen eines zeitlich veränderlichen elektromagnetischen Feldes mittels einer Folge von Stromimpulsen**
Magnetic field therapy device for generating a time varying electromagnetic field by means of a train of current pulses
Appareil de thérapie magnétique pour générer un champ magnétique variable à l'aide de trains d'impulsions de courant

(43) Veröffentlichungstag der Anmeldung: 22.11.2006
(73) Patentinhaber: Medithera Medizinische Systeme AG, 56462 Höhn (DE)
(72) Erfinder: Öhlenschläger, Manfred, Dipl.-Ing., 64668 Rimbach (DE)
(74) Vertreter: Schmidt, Frank-Michael

(56) Entgegenhaltungen:
- WO-A-03/103769
- WO-A-20/04087255
- US-A- 3 678 337
- US-A1- 2002 165 583
- US-B1- 6 858 000

## Beschreibung

Die Erfindung betrifft ein Magnetfeldtherapiegerät, das einen Impulsgenerator mit einer Stromtreiberendstufe und eine mit der Stromtreiberendstufe verbundene Feldspule zum Erzeugen eines elektromagnetischen Feldes, welches auf eine zu behandelnde Körperregion einwirkt, aufweist, wobei der Impulsgenerator mit der Stromtreiberendstufe derart ausgebildet ist, daß in der Feldspule ein Stromfluß erzeugt wird, der aus einer Folge mehrerer Wiederholungen einer Impulssequenz besteht, wobei die Impulssequenz aus mehreren Stromimpulsen gleicher Grundform besteht, die eine variierende Amplitude und eine variierende Impulsbreite haben können und durch variierende Impulspausen voneinander beabstandet sind.

Eine Vorrichtung der eingangs genannten Art ist beispielsweise aus der Patentschrift EP 0 594 655 B1 bekannt. Die bekannte Vorrichtung erzeugt einen speziellen Stromverlauf, nämlich eine Folge von Stromimpulsen, mit deren Hilfe der Transport von Ionen, insbesondere von Protonen (H⁺), in Körperbereichen bei Menschen und Tieren zur gezielten Beeinflussung von Ionenkonzentrationen ermöglicht werden soll. Durch die in Körperflüssigkeiten, wie Blut oder Lymphe, durch das sich ändernde Magnetfeld induzierten Spannungen und bewirken Stromflüsse soll ein gezielter Ionentransport über von Gefäßwänden gebildeten Potentialbarrieren hinweg ermöglicht werden. Die Druckschrift schlägt deshalb vor, daß die in einer vorzugsweise induktionsarm ausgebildeten Feldspule erzeugten gepulsten Ströme folgende Eigenschaften aufweisen: (a) ein Grundstromimpuls besteht aus einer Überlagerung eines Rechteck-Stroms und eines etwa nach einer e-Funktion ansteigenden Stroms, gefolgt von einer wenigstens gleich langen Impulspause; (b) die Grundfrequenz der Grundstromimpulse mit Grundimpulspausen beträgt 100 bis 1000 Hz, vorzugsweise 200 Hz; (c) die Amplitude der Grundimpulsfolge ist mit einer Modulationsfrequenz von 0,5 bis 25 Hz, vorzugsweise 20 Hz, in der Amplitude moduliert; und (d) die modulierte Grundimpulsfolge wird als Impulsfolgeserie für eine Zeitdauer von 0,3 bis 1,0 s ausgesendet, woran sich jeweils eine Impulsserienpause von 0,7 bis 5 s anschließt.

Daneben sind im Stand der Technik weitere Magnetfeldtherapiegeräte bekannt, bei denen mit Hochfrequenzschwingungen überlagerte Rechteckimpulse verwendet werden.

Aus der US 2002/0165583 A1 ist ein Magnetfeldtherapiegerät zur Behandlung von Osteoporose und Stimulation von Gewebewachstum bekannt, bei dem Folgen asymmetrischer Rechteckimpulse in Bursts von jeweils 1609 Impulsen angewendet werden. Ein weiteres Gerät, welches elektromagnetische Felder erzeugt, ist in US 3,678,337A offenbart.

Aufgabe der Erfindung ist es, die Wirksamkeit der genannten Magnetfeldtherapiegeräte zu verbessern.

Diese Aufgabe wird erfindungsgemäß durch ein Magnetfeldtherapiegerät mit den Merkmalen des Anspruchs 1 gelöst.

Erfindungsgemäß ist ein Magnetfeldtherapiegerät der eingangs genannten Art dadurch gekennzeichnet, daß bei jedem Stromimpuls der Betrag des Stromes ausgehend von einem Anfangswert und beginnend mit einem ersten Anstieg ansteigt, wobei sich der Anstieg stetig bis zu einem zweiten Anstieg erhöht und dann stetig bis zu einem dritten Anstieg abfällt, woraufhin der Betrag des Stromes sprunghaft wieder auf den Anfangswert abfällt. Unter einem sprunghaften Abfall soll hierbei ein steiler Abfall eines Stromes durch eine Spule verstanden werden, soweit dieser innerhalb der physikalischen Grenzen möglich ist. Vorzugsweise erhöht sich der Anstieg zwischen dem ersten und dem zweiten Anstieg linear und fällt dann linear auf den dritten Anstieg ab. Vorzugsweise sind sowohl der Anfangswert als auch der erste und der dritte Anstieg etwa gleich Null. Es hat sich gezeigt, daß mit der beschriebenen Grundimpulsform in Verbindung mit geeigneten Impulssequenzen eine deutliche Verbesserung der therapeutischen Wirkungen erzielbar ist. Bei der gewählten Impulsform ist es möglich, durch geeignete Gestaltung der Impulssequenz innerhalb des Spektralbereichs des erzeugten Magnetfelds bestimmte erwünschte Frequenzanteile hervorzuheben und unerwünschte Frequenzanteile zu unterdrücken.

Bei einer bevorzugten Weiterbildung des erfindungsgemäßen Magnetfeldtherapiegeräts schließt sich jedem Impuls der Impulssequenz eine Impulspause an, in der der Strombetrag dem Anfangswert entspricht (vorzugsweise gleich Null ist), wobei sich vorzugsweise - in jeder Impulssequenz - wenigstens einer Folge kurzer Impulspausen wenigstens eine längere Impulspause anschließt, so daß in der Impulssequenz Gruppen von Impulsen gebildet werden. Die Impulsbreite liegt beispielsweise zwischen 0,5 und 150 ms, vorzugsweise bei etwa 2 ms. Die Impulspausen können eine Dauer von bis zu 10 s haben. Vorzugsweise werden die Breiten der sich jedem Impuls anschließenden Impulspausen variiert, indem nach jedem Impuls eine von mehreren vorgegebenen Impulspausen fester Dauer ausgewählt wird. Beispielsweise sind 2 bis 10, vorzugsweise 4, Impulspausen unterschiedlicher Dauer vorgegeben, aus denen jeweils eine ausgewählt wird. Beispielsweise werden eine Anzahl von 3 bis 10 Stromimpulse mit der kürzesten Impulspause (die vorzugsweise etwa der Dauer des Impulses entspricht) hintereinander angeordnet, woran sich eine längere Impulspause anschließt, woraufhin wiederum eine Gruppe von 3 bis 10 Impulsen mit kurzen Impulspausen folgt. Nachdem beispielsweise 3 bis 6 derartige Gruppen hintereinander erzeugt worden sind, folgt eine noch längere Impulspause. Dies wird einige Male wiederholt und ergibt die vollständige Impulssequenz, welche dann während der Behandlungsdauer mehrfach wiederholt angewendet wird. Bei einer alternativen Ausführungsform könnte die Impulssequenz auch erzeugt werden, indem die Impulspausen nach einer weniger systematischen als vielmehr zufälligen Sequenz gewählt werden, wobei allerdings die Impulspausenfolge so gewählt wird, daß sich insgesamt ein vorgegebenes Signalspektrum ergibt. Durch geeignete Wahl und Anordnung der Impulspausen werden insbesondere Spektralanteile im Frequenzbereich zwischen 0,3 Hz und 1 kHz eingestellt. Die Auswahl der Impulspausen aus einer vorgegebenen geringen Anzahl möglicher Impulspausen vereinfacht die Steuerung und den schaltungstechnischen Aufwand für die Erzeugung der Impulssequenz.

Bei einer bevorzugten Weiterbildung des erfindungsgemäßen Magnetfeldtherapiegeräts werden die Impulsamplitude und/oder die Impulsbreite innerhalb der Impulssequenz nach einem vorgegebenen Schema moduliert. Beispielsweise kann die Impulsamplitude innerhalb einer Gruppe von Impulsen der Impulssequenz so moduliert werden, daß die Impulsamplitude vom Beginn bis zu Mitte der Gruppe ansteigt und danach wieder abfällt. Bei einer Modulation der Impulsbreite ist es beispielsweise möglich, daß die Impulsbreite und die Dauer der jeweils zugehörigen Impulspause zumindest in einem Abschnitt der Impulssequenz (beispielsweise innerhalb einer Gruppe) so variiert werden, daß das Tastverhältnis, das heißt das Verhältnis zwischen Impulsbreite und Breite der Impulspause, gleich bleibt. Diese zusätzlichen Möglichkeiten der Einflußnahme auf den Signalverlauf innerhalb der Impulssequenz gestatten erweiterte Möglichkeiten der Einflußnahme auf die spektrale Zusammensetzung, erhöhen aber gleichzeitig den baulichen und steuerungstechnischen Aufwand.

Bei einer bevorzugten Ausführungsform des Magnetfeldtherapiegeräts enthält der Impulsgenerator einen Mikrocontroller mit einem Digital-Analog-Umsetzer, der eine Steuerspannung für die Stromtreiberendstufe erzeugt. Die Betriebsfrequenz des Mikrocontrollers ist dabei so hoch gewählt (beispielsweise im Bereich von 10 MHz bis 100 MHz), daß die oben genannten Signalverläufe für die mit Hilfe des Digital-Analog-Umsetzers erzeugte Steuerspannung programmgesteuert realisiert werden können.

Weitere vorteilhafte und/oder bevorzugte Ausführungsformen des erfindungsgemäßen Magnetfeldtherapiegeräts sind in den Unteransprüchen gekennzeichnet.

Im folgenden wird die Erfindung anhand bevorzugter Ausführungsformen näher beschrieben, die anhand der Zeichnungen veranschaulicht werden. In den Zeichnungen zeigen:
Figur 1 ein Blockschaltbild eines Magnetfeldtherapiegeräts;
Figur 2 eine schematische Darstellung der Anwendung des Magnetfeldtherapiegeräts;
Figur 3 eine Darstellung des bei dem erfindungsgemäßen Magnetfeldtherapiegerät verwendeten Grundstromimpulses;
Figur 4 ein Strom-Zeit-Diagramm, das ein Beispiel einer Impulspausenmodulation zeigt;
Figur 5 ein Strom-Zeit-Diagramm, das ein Beispiel einer Amplitudenmodulation veranschaulicht;
Figur 6 ein Strom-Zeit-Diagramm, das ein Beispiel einer Frequenzmodulation veranschaulicht; und
Figur 7 ein Strom-Zeit-Diagramm einer beispielhaften Impulssequenz.

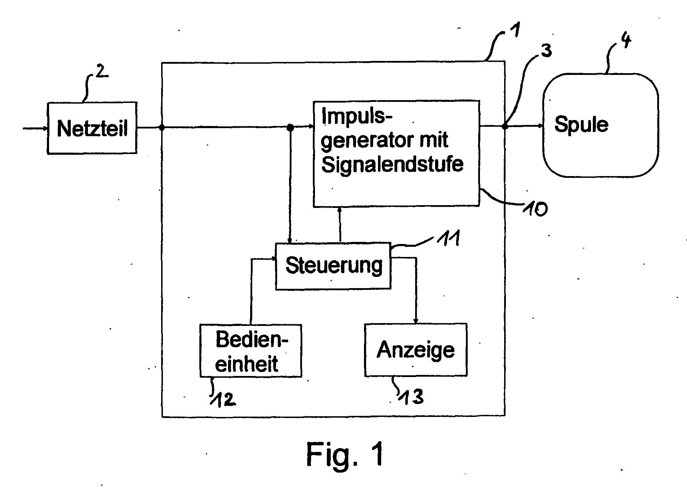

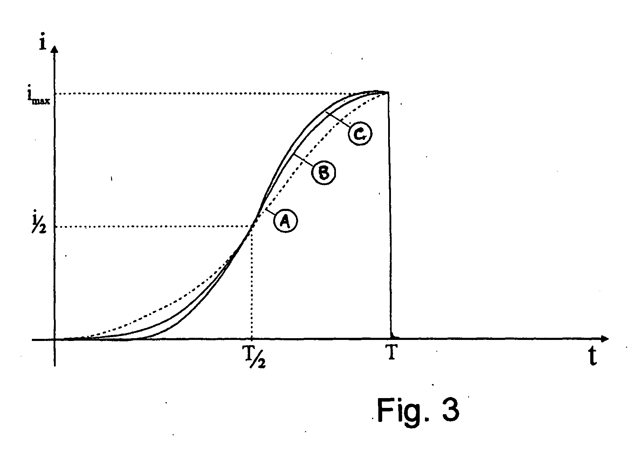

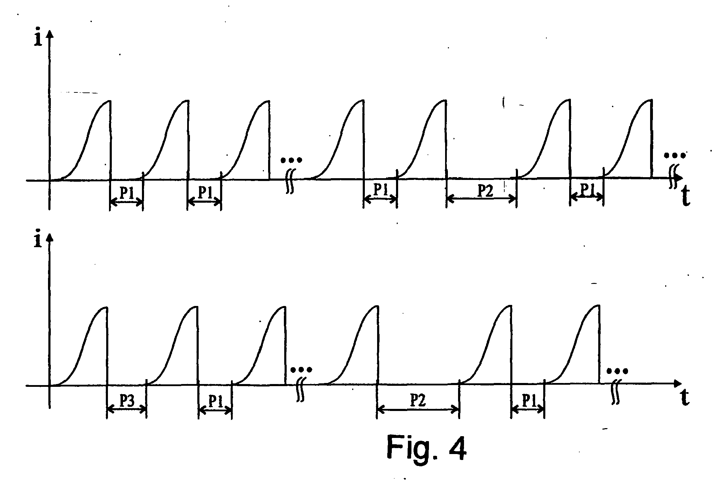

Fig. 1 zeigt ein Blockschaltbild eines Magnetfeldtherapiegeräts. In einem Gehäuse 1 sind ein Impulsgenerator 10 und eine den Impulsgenerator steuernde Steuerschaltung 11 mit zugeordneter Bedieneinheit 12 und Anzeigeeinheit 13 untergebracht. Ein Netzteil 2 sichert die Spannungsversorgung der Schaltungen des Magnetfeldtherapiegeräts. Ein Ausgangsanschluß 3 des Impulsgenerators 10 ist aus dem Gehäuse herausgeführt. An dem Ausgang 3 wird eine Feldspule 4 angeschlossen. Der Impulsgenerator 10 enthält eine Stromtreiberendstufe (auch als Signalendstufe bezeichnet), die über den Ausgang 3 den durch die Spule 4 fließenden Strom zur Verfügung stellt. Der Impulsgenerator 10 enthält eine Schaltungsanordnung, die ein an einem Eingang des Impulsgenerators eingegebenes Spannungssignal in ein entsprechendes Stromsignal umsetzt. Das Spannungssignal wird dem Impulsgenerator 10 von der Steuerschaltung 11 zur Verfügung gestellt. Die Steuerschaltung 11 erzeugt das Spannungssignal und legt somit den zeitlichen Verlauf des Stromflusses durch die Spule 4 fest. Die Steuerschaltung 11 erzeugt das Spannungssignal mit Hilfe eines Digital-Analog-Umsetzers. Die dem Digital-Analog-Umsetzer eingegebenen Digitalwerte werden programmgesteuert von einem Controller erzeugt. Bei einer alternativen Ausführungsform des Magnetfeldtherapiegeräts kann die Steuerschaltung 11 auch mehrere Spannungssignale erzeugen, die einer entsprechenden Anzahl von Impulsgeneratoren eingegeben werden, so daß gleichzeitig mehrere Feldspulen angesteuert werden können. Zum Programmieren oder Einstellen des gewünschten Stromsignalverlaufs ist die Steuerschaltung 11 mit einer Bedieneinheit 12 und einer Anzeige 13 versehen. Über eine entsprechende Bedienerführung auf der Anzeige 13 wird ein Bediener des Magnetfeldtherapiegeräts bei seinen Eingaben in die Bedieneinheit 12 unterstützt. In Abhängigkeit von den Bedienereingaben legt die Steuerschaltung einen zeitlichen Verlauf des Stromflusses fest, der aus einer Folge mehrerer Wiederholungen einer Impulssequenz besteht. Die mehrfach wiederholte Impulssequenz besteht aus mehreren Stromimpulsen gleicher Grundform. Die Grundform der Stromimpulse wird unten näher anhand von Fig. 3 beschrieben. Die Steuerschaltung legt in Abhängigkeit von Bedienereingaben und von abgearbeiteten Programmen fest, in welchen Abständen, welcher Amplitude und mit welcher Impulsbreite die Stromimpulse in der Impulssequenz aufeinander folgen. Dies wird unten näher anhand der Fig. 4 bis 7 erläutert.

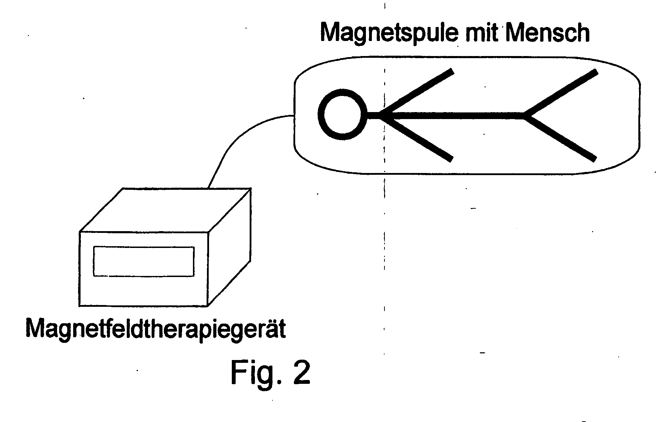

Fig. 2 zeigt eine Anwendung des Magnetfeldtherapiegeräts, bei der eine zu behandelnde Person auf einer flexiblen Matte liegt, in der eine Feldspule (auch als Magnetspule bezeichnet) eingebettet ist, welche aus mehreren in der Ebene der flexiblen Matte liegenden Spulenwindungen besteht. Vorzugsweise umfaßt die Spule relativ wenige Windungen (und hat somit eine geringe Induktivität), die nebeneinander spiralförmig in die Matte eingebracht sind. Die Größe der Windungen entspricht der Größe der zu behandelnden Körperregion. Es können auch mehrere Spulen mit jeweils mehreren Wicklungen nebeneinander in der Matte untergebracht sein, wobei die Spulen entweder hintereinander geschaltet oder separat angesteuert werden können. Eine typische Magnetspule für das erfindungsgemäße Magnetfeldtherapiegerät enthält zwischen 10 und 100 Windungen. Die Breite und Dicke der Spulenleitbahnen ist ausreichend groß, um einen geringen ohmschen Widerstand zur Verfügung zu stellen.

Fig. 3 zeigt ein Strom-Zeit-Diagramm, das den Stromverlauf einiger bevorzugter Stromimpulse darstellt. Die drei mit den Buchstaben A, B und C gekennzeichneten Beispiel-Stromverläufe weisen folgende Gemeinsamkeiten auf: Zum Zeitpunkt t = 0 ist der Strom i ebenfalls gleich 0. Mit zunehmender Zeit t steigt der Strom i an, wobei der Stromanstieg zu Beginn ebenfalls gleich 0 ist, dann stetig bis zum Zeitpunkt T/2 ansteigt, wobei der Strom i zum Zeitpunkt T/2 die Hälfte seines Maximalwerts iₘₐₓ erreicht. Anschließend steigt der Strom i weiterhin an, wobei aber der Anstieg des Stromes abfällt, bis er zum Zeitpunkt t = T wieder den Wert 0 erreicht, wobei der Strom i gleichzeitig den Maximalwert iₘₐₓ erreicht. Zum Zeitpunkt T fällt der Strom i dann sprunghaft auf den Wert 0 ab. Selbstverständlich ist dieser Abfall aufgrund der (geringen) Induktivität der Spule sowie aufgrund der der Treiberschaltung innewohnenden parasitären Widerstände und Kapazitäten nur näherungsweise sprunghaft, da die im Magnetfeld gespeicherte Energie eine sprunghafte Stromänderung nicht erlaubt.

Der Anstieg des Stroms i nimmt im Intervall [0; T/2] stetig vorzugsweise linear, zu und fällt im Intervall [T/2; T] stetig, vorzugsweise linear, ab. Der Stromverlauf des Grundimpulses führt zu einer Induktionsspannung, die im Intervall [0; T/2] stetig, vorzugsweise linear, ansteigt und im Intervall [T/2; T] stetig, vorzugsweise linear, abfällt. Der sprunghafte Abfall des Stroms i zum Zeitpunkt T führt zu einer kurzen Induktionsspannungsspitze umgekehrter Polarität, deren Dauer wesentlich geringer ist als die Dauer (und Wirkzeit) des Induktionsspannungsimpulses der Dauer T.

Stromimpulse mit der in Fig. 3 dargestellten Grundform werden nunmehr in vorgegebenen Abständen wiederholt, wobei sowohl die Amplitude der Stromimpulse als auch deren Impulsbreite sowie die zwischen den Impulsen befindlichen Impulspausen variiert werden können. Die definierte Aneinanderreihung mehrerer Stromimpulse bildet die Impulssequenz, wobei eine vorgegebene Impulssequenz während der Behandlungsdauer von mehreren Minuten mehrfach wiederholt wird.

Fig. 4 zeigt ein Strom-Zeit-Diagramm von Abschnitten einer Impulssequenz, wobei sich dem oberen Strom-Zeit-Verlauf der untere Strom-Zeit-Verlauf anschließt. Die in Fig. 4 dargestellte Impulssequenz wurde mittels einer Impulspausenmodulation, d.h. einem Variieren der Impulspausen zwischen benachbarten Stromimpulsen gleicher Impulsamplitude und gleicher Impulsbreite, gewonnen. Bei dem in Fig. 4 dargestellten Beispiel gibt es drei verschiedene Impulspausen P1, P2 und P3 unterschiedlicher Länge mit P1 < P3 < P2.

Bei der Impulspausenmodulation, wie sie in Fig. 4 dargestellt ist, werden vorzugsweise eine begrenzte Anzahl (3 bis 10, vorzugsweise 4) unterschiedliche Impulspausen vorab definiert. Anschließend wird die Impulssequenz zusammengestellt, indem jedem Stromimpuls eine nachfolgende Impulspause durch Auswahl aus einer der vorab definierten Impulspausen zugeordnet wird.

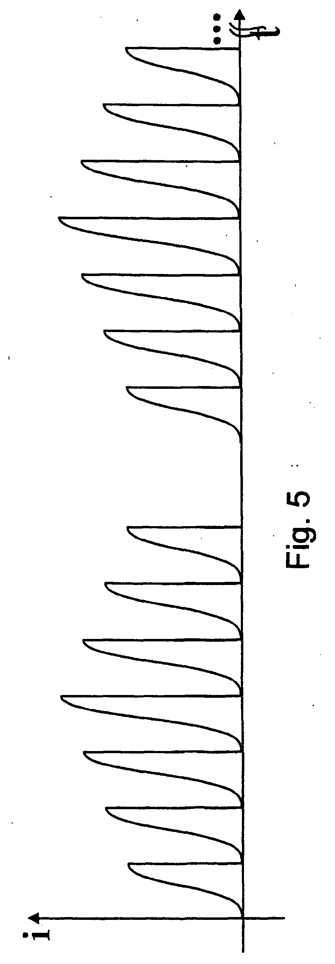

Fig. 5 zeigt schematisch eine weitere Möglichkeit der Modulation der Impulssequenz, bei der nicht der Abstand oder die Breite der Stromimpulse variiert werden, sondern deren Höhe (Amplitude). Ausgehend von einer vorgegebenen maximalen Impulshöhe kann die aktuelle Impulshöhe eines auszugebenen Impulses als Prozentwert des Maximalwerts dargestellt werden, wobei eine Amplitude zwischen 0 und 100 % gewählt werden kann. Alternativ kann die Amplitudenmodulation auch durch Überlagerung (Addition) einer vorgegebenen Anzahl gleicher Stromimpulse bewirkt werden.

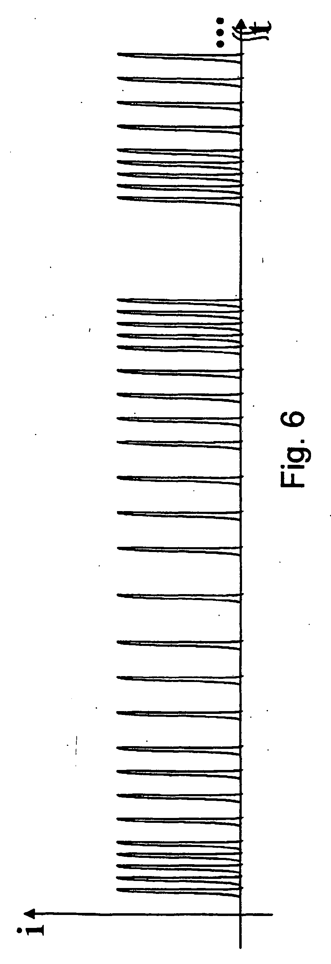

Fig. 6 zeigt eine dritte Möglichkeit der Modulation der Impulssequenz, bei der nicht die Impulshöhe (Amplitude), sondern der Impulsabstand (und ggf. gleichzeitig die Impulsbreite) moduliert wird. Dies stellt eine Frequenzmodulation dar.

Die in den Fig. 4 bis 6 gezeigten beispielhaften Modulationsarten können auch kombiniert eingesetzt werden.

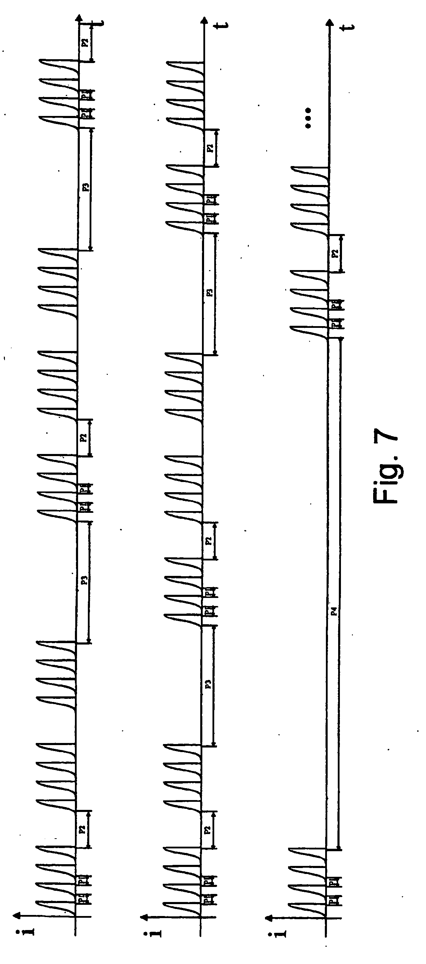

Fig. 7 zeigt eine bevorzugte Ausführungsform einer erfindungsgemäßen Impulssequenz, welche im obersten Strom-Zeit-Diagramm beginnt und sich bis zum untersten Strom-Zeit-Diagramm fortsetzt. Bei der Impulssequenz werden zunächst Gruppen von vier Stromimpulsen, die durch eine kurze Impulspause P1 beabstandet sind, ausgegeben. Jeweils drei aufeinanderfolgende Vier-Stromimpuls-Gruppen sind wiederum durch eine etwas längere Impulspause P2 beabstandet. Nach den drei Vier-Stromimpuls-Gruppen folgt dann eine längere Impulspause P3. Nachdem fünfmal, jeweils beabstandet durch die Pause P3, drei Vier-Stromimpuls-Gruppen ausgegeben worden sind (d.h. insgesamt vier x drei x fünf = sechzig Stromimpulse), folgt die längste Impulspause P4. Anschließend werden die sechzig Stromimpulse wiederholt.

Durch diese anhand des Beispiels gemäß Fig. 7 veranschaulichte Bildung von Impulsgruppen und Impulsuntergruppen läßt sich ein Frequenzspektrum der Impulssequenz gezielt einstellen, bei dem bestimmte Frequenzanteile hervorgehoben und andere Frequenzanteile unterdrückt werden. Insbesondere können durch geeignete Gestaltung der Impuls-Impulspause-Folgen Frequenzen im Bereich zwischen 0,3 Hz und 1 kHz mittels einfacher Steueralgorithmen gezielt angehoben oder unterdrückt werden. Die relativ einfache Technik des Auswählens aus einer geringen Anzahl vorgegebener Impulspausen gestattet eine einfache und dennoch flexible Einstellung gewünschter Spektren des veränderlichen Magnetfelds.

Die erfindungsgemäße Ausgestaltung der Impulssequenz ermöglicht zweierlei Optimierungen. Zum einen kann die Stromimpulssequenz derart gestaltet werden, daß die Stimulation der biologischen Zellen mit gewünschten Frequenzanteilen erfolgt. Zellen reagieren auf bestimmte stimulierende Frequenzen bzw. Frequenzmuster positiv. Zum anderen können Frequenzen bzw. Frequenzmuster unterdrückt werden, die negative Einflüsse auf biologische Zellen haben. Beispielsweise sind Frequenzanteile im Bereich 50 Hz unerwünscht; deren negativer Einfluß auf Zellen ist bereits untersucht worden.

## Patentansprüche

1. Magnetfeldtherapiegerät, das einen Impulsgenerator (10) mit einer Stromtreiberendstufe und eine mit der Stromtreiberendstufe verbundene Feldspule (4) zum Erzeugen eines elektromagnetischen Feldes, welches auf eine zu behandelnde Körperregion einwirkt, aufweist,
wobei der Impulsgenerator (10) mit der Stromtreiberendstufe derart ausgebildet ist, daß in der Feldspule (4) ein Stromfluß erzeugt wird, der aus einer Folge mehrerer Wiederholungen einer Impulssequenz besteht,
wobei die Impulssequenz aus mehreren Stromimpulsen gleicher Grundform besteht, die eine variierende Amplitude und eine variierende Impulsbreite haben können und durch variierende Impulspausen voneinander beabstandet sein können,
**dadurch gekennzeichnet,**
**daß** bei jedem Stromimpuls der Betrag des Stromes ausgehend von einem Anfangswert und beginnend mit einem ersten Anstieg ansteigt, wobei sich der Anstieg stetig bis zu einem zweiten Anstieg erhöht und dann stetig bis zu einem dritten Anstieg abfällt, woraufhin der Betrag des Stromes sprunghaft wieder auf den Anfangswert abfällt.

2. Magnetfeldtherapiegerät nach Anspruch 1, **dadurch gekennzeichnet, daß** sich der Anstieg linear vom ersten zum zweiten Anstieg erhöht und dann linear auf den dritten Anstieg abfällt.

3. Magnetfeldtherapiegerät nach Anspruch 1 oder 2, **dadurch gekennzeichnet, daß** sich der Anstieg ausgehend von dem ersten Anstieg stetig für eine vorgegebene Zeitdauer erhöht, so daß der zweite Anstieg erreicht wird.

4. Magnetfeldtherapiegerät nach einem der Ansprüche 1 bis 3, **dadurch gekennzeichnet, daß** sich jedem Impuls in der Impulssequenz eine Impulspause anschließt, in der der Strombetrag dem Anfangswert entspricht.

5. Magnetfeldtherapiegerät nach Anspruch 4, **dadurch gekennzeichnet, daß** sich in jeder Impulssequenz wenigstens einer Folge kurzer Impulspausen wenigstens eine längere Impulspause anschließt, so daß in der Impulssequenz Gruppen von Impulsen gebildet werden.

6. Magnetfeldtherapiegerät nach einem der Ansprüche 1 bis 5, **dadurch gekennzeichnet, daß** der Anfangswert gleich Null ist.

7. Magnetfeldtherapiegerät nach einem der Ansprüche 1 bis 6, **dadurch gekennzeichnet, daß** der erste Anstieg und der dritte Anstieg näherungsweise gleich Null sind.

8. Magnetfeldtherapiegerät nach einem der Ansprüche 1 bis 7, **dadurch gekennzeichnet, daß** die Impulsbreite zwischen 0,5 und 150 ms liegt, vorzugsweise etwa 2 ms beträgt.

9. Magnetfeldtherapiegerät nach Anspruch 8, **dadurch gekennzeichnet, daß** die Impulspausen eine Dauer von bis zu 10 s haben.

10. Magnetfeldtherapiegerät nach Anspruch 8 oder 9, **dadurch gekennzeichnet, daß** die Impulssequenz eine Dauer zwischen 0,002 und 20s aufweist und für eine Behandlungsdauer zwischen 1 und 99min wiederholt wird.

11. Magnetfeldtherapiegerät nach einem der Ansprüche 1 bis 10, **dadurch gekennzeichnet, daß** die Breiten der sich jedem Impuls anschließenden Impulspausen variiert werden, indem nach jedem Impuls eine von mehreren vorgegebenen Impulspausen fester Dauer ausgewählt wird.

12. Magnetfeldtherapiegerät nach Anspruch 11, **dadurch gekennzeichnet, daß** 2 bis 10, vorzugsweise 4, Impulspausen verschiedener Dauer vorgegeben werden.

13. Magnetfeldtherapiegerät nach einem der Ansprüche 1 bis 12, **dadurch gekennzeichnet, daß** die Impulsamplitude und/oder die Impulsbreite innerhalb der Impulssequenz nach einem vorgegebenen Schema moduliert werden.

14. Magnetfeldtherapiegerät nach Anspruch 13, **dadurch gekennzeichnet, daß** die Impulsbreite und die Dauer der jeweils zugehörigen Impulspause zumindest in einem Abschnitt der Impulssequenz so variiert werden, daß das Tastverhältnis gleich bleibt.

15. Magnetfeldtherapiegerät nach einem der Ansprüche 1 bis 14, **dadurch gekennzeichnet, daß** die Impulsamplituden, Impulsbreiten und/oder Impulspausen innerhalb der Impulssequenz so vorgegeben werden, daß ein vorgegebenes Spektrum der Frequenzanteile des Stromes erzielt wird, bei dem erwünschte Frequenzanteile hervorgehoben werden.

16. Magnetfeldtherapiegerät nach einem der Ansprüche 1 bis 15, **dadurch gekennzeichnet, daß** die Impulsamplituden, Impulsbreiten und/oder Impulspausen in der Impulsfrequenz so vorgegeben werden, daß unerwünschte Frequenzanteile unterdrückt werden.

## Claims

1. Magnetic field therapy device which has a pulse generator (10) with a current driver output stage and a field coil (4) connected to the current driver output stage in order to generate an electromagnetic field which acts on a region of the body which is to be treated, wherein the pulse generator (10) with the current driver output stage is constructed in such a way that in the field coil (4) a current flow is produced which consists of a train of several repetitions of a pulse sequence, wherein the pulse sequence consists of a plurality of current pulses of the same basic shape which can have a varying amplitude and a varying pulse width and can be spaced from one another by varying pulse intervals, **characterised in that** with each current pulse the magnitude of the current increases starting from an initial value and beginning with a first increase, wherein the increase rises continuously up to a second increase and then falls continuously to a third increase, whereupon the magnitude of the current falls suddenly again to the initial value.

2. Magnetic field therapy device as claimed in Claim 1, **characterised in that** the increase rises linearly from the first to the second increase and then falls linearly to the third increase.

3. Magnetic field therapy device as claimed in Claim 1 or 2, **characterised in that** the increase rises continuously starting from the first increase for a predetermined period of time so that the second increase is reached.

4. Magnetic field therapy device as claimed in any one of Claims 1 to 3, **characterised in that** a pulse interval in which the current magnitude corresponds to the initial value follows each pulse in the pulse sequence.

5. Magnetic field therapy device as claimed in Claim 4, **characterised in that** in each pulse sequence at least one longer pulse interval follows at least one train of short pulse intervals, so that groups of pulses are formed in the pulse sequence.

6. Magnetic field therapy device as claimed in any one of Claims 1 to 5, **characterised in that** the initial value is equal to zero.

7. Magnetic field therapy device as claimed in any one of Claims 1 to 6, **characterised in that** the first increase and the third increase are approximately equal to zero.

8. Magnetic field therapy device as claimed in any one of Claims 1 to 7, **characterised in that** the pulse width is between 0.5 and 150 ms, and is preferably approximately 2 ms.

9. Magnetic field therapy device as claimed in Claim 8, **characterised in that** the pulse intervals have a duration of up to 10 s.

10. Magnetic field therapy device as claimed in Claim 8 or 9, **characterised in that** the pulse sequence has a duration between 0.002 and 20 s and is repeated for a treatment duration between 1 and 99 min.

11. Magnetic field therapy device as claimed in any one of Claims 1 to 10, **characterised in that** the widths of the pulse intervals following each pulse are varied **in that** after each pulse one of several predetermined pulse intervals of fixed duration are selected.

12. Magnetic field therapy device as claimed in Claim 11, **characterised in that** 2 to 10, preferably 4, pulse intervals of different duration are predetermined.

13. Magnetic field therapy device as claimed in any one of Claims 1 to 12, **characterised in that** the pulse amplitude and/or the pulse width within the pulse sequence are modulated according to a predetermined scheme.

14. Magnetic field therapy device as claimed in Claim 13, **characterised in that** the pulse width and the duration of the respective associated pulse interval are varied at least in a portion of the pulse sequence so that the keying ratio remains the same.

15. Magnetic field therapy device as claimed in any one of Claims 1 to 14, **characterised in that** the pulse amplitudes, pulse widths and/or pulse intervals within the pulse sequence are predetermined so that a predetermined spectrum of the frequency components of the current is achieved at which desired frequency components are emphasised.

16. Magnetic field therapy device as claimed in any one of Claims 1 to 15, **characterised in that** the pulse amplitudes, pulse widths and/or pulse intervals in the pulse frequency are predetermined in such a way that undesired frequency components are suppressed.

## Revendications

1. Appareil de thérapie à champ magnétique, qui présente un générateur d'impulsions (10) avec un étage de sortie à excitation de courant et une bobine de champ (4) connectée à l'étage de sortie à excitation de courant pour générer un champ électromagnétique, qui exerce une action sur une région du corps à traiter,
dans lequel le générateur d'impulsions (10) avec l'étage de sortie à excitation de courant est conçu de manière à générer dans la bobine de champ (4) un flux de courant qui soit constitué d'une série de plusieurs répétitions d'une séquence d'impulsions,
laquelle séquence d'impulsions est formée de plusieurs impulsions de courant de même forme de base, qui peuvent avoir une amplitude variable et une largeur d'impulsion variable et qui peuvent être espacées les unes des autres par des pauses d'impulsions variables,
**caractérisé en ce que**,
à chaque impulsion de courant, la valeur du courant augmente en partant d'une valeur initiale et en commençant par une première augmentation, laquelle augmentation s'élève en continu jusqu'à une deuxième augmentation, puis diminue en continu jusqu'à une troisième augmentation, à la suite de quoi la valeur du courant redescend brusquement à la valeur initiale.

2. Appareil de thérapie à champ magnétique selon la revendication 1, **caractérisé en ce que** l'augmentation s'élève de manière linéaire de la première à la deuxième augmentation, puis diminue de manière linéaire jusqu'à la troisième augmentation.

3. Appareil de thérapie à champ magnétique selon la revendication 1 ou 2, **caractérisé en ce que** l'augmentation s'élève, en partant de la première augmentation, de façon continue pendant une durée prédéfinie de manière à atteindre la deuxième augmentation.

4. Appareil de thérapie à champ magnétique selon l'une quelconque des revendications 1 à 3, **caractérisé en ce que**, à chaque impulsion de la séquence d'impulsions, succède une pause d'impulsions, dans laquelle la valeur du courant correspond à la valeur initiale.

5. Appareil de thérapie à champ magnétique selon la revendication 4, **caractérisé en ce que**, à chaque séquence d'impulsions, au moins une pause d'impulsions plus longue succède à au moins une suite de pauses d'impulsion courtes de manière à former dans la séquence d'impulsions des groupes d'impulsions.

6. Appareil de thérapie à champ magnétique selon l'une quelconque des revendications 1 à 5, **caractérisé en ce que** la valeur initiale est égale à zéro.

7. Appareil de thérapie à champ magnétique selon l'une quelconque des revendications 1 à 6, **caractérisé en ce que** la première augmentation et la troisième augmentation sont approximativement égales à zéro.

8. Appareil de thérapie à champ magnétique selon l'une quelconque des revendications 1 à 7, **caractérisé en ce que** la largeur d'impulsion se situe entre 0,5 et 150 ms, et est de préférence d'environ 2 ms.

9. Appareil de thérapie à champ magnétique selon la revendication 8, **caractérisé en ce que** les pauses d'impulsions ont une durée allant jusqu'à 10 s.

10. Appareil de thérapie à champ magnétique selon la revendication 8 ou 9, **caractérisé en ce que** la séquence d'impulsions présente une durée comprise entre 0,002 et 20 s et est répétée pour une durée de traitement de 1 à 99 mn.

11. Appareil de thérapie à champ magnétique selon l'une quelconque des revendications 1 à 10, **caractérisé en ce que** l'on fait varier les largeurs des pauses d'impulsions succédant à chaque impulsion en choisissant, après chaque impulsion, une durée fixe de plusieurs pauses d'impulsions prédéfinies.

12. Appareil de thérapie à champ magnétique selon la revendication 11, **caractérisé en ce que** l'on fixe 2 à 10, de préférence 4, pauses d'impulsions de durée différente.

13. Appareil de thérapie à champ magnétique selon l'une quelconque des revendications 1 à 12, **caractérisé en ce que** l'amplitude d'impulsion et/ou la largeur d'impulsion dans la séquence d'impulsions est/sont modulées selon un schéma prédéfini.

14. Appareil de thérapie à champ magnétique selon la revendication 13, **caractérisé en ce que** la largeur d'impulsion et la durée de la pause d'impulsions respectivement correspondante varient au moins dans une section de la séquence d'impulsions, de sorte que le taux d'impulsions reste identique.

15. Appareil de thérapie à champ magnétique selon l'une quelconque des revendications 1 à 14, **caractérisé en ce que** l'on prédéfinit les amplitudes d'impulsions, les largeurs d'impulsions et/ou les pauses d'impulsions dans la séquence d'impulsions de manière à obtenir un spectre prédéfini des portions de fréquences du courant, dans lequel les portions de fréquences souhaitées sont mises en évidence.

16. Appareil de thérapie à champ magnétique selon l'une quelconque des revendications 1 à 15, **caractérisé en ce que** l'on prédéfinit les amplitudes d'impulsions, les largeurs d'impulsions et/ou les pauses d'impulsions dans la séquence d'impulsions de manière à supprimer les portions de fréquences non souhaitées.
